# EUROPEAN PATENT APPLICATION

(11) **EP 4 248 959 A1**
(43) Date of publication of application: **27.09.2023**
(21) Application number: 22163450.4
(22) Date of filing: 22.03.2022
(51) Int. Cl.: A61K 31/16, A61P 43/00

(54) **PREPARATION FOR REDUCTION OF PATHOLOGICAL IRON OVERLOAD**

(71) Applicant: Dr. Franz Köhler Chemie GmbH, 64625 Bensheim (DE)
(72) Inventor: KÖHLER, Gernot, 65665 Alsbach-Hähnlein (DE); KÖHLER, Anita, 65665 Alsbach-Hähnlein (DE)
(74) Representative: Blumbach · Zinngrebe Patentanwälte PartG mbB

(57) **Abstract**

The invention relates to a preparation for reduction of pathological iron overload in human body, especially for treatment of hemochromatosis. The preparation comprising a hydroxamic acid derivate, wherein the hydroxamic acid derivate builds a complex with iron ions, wherein the hydroxamic derivate comprises a hydroxamic acid group and wherein the hydrogen atom on the hydroxamic acid carbon is aryl- or alkylaryl substituted, wherein said substituent may comprise hydroxy, alkoxy or amino groups.

## Description

### State of the art

The invention at hand relates to a preparation for reduction of pathological iron overload in human body, especially for treatment of hemochromatosis.

Haemochromatosis results from chronic iron overload, which can manifest symptomatically, especially in the parenchymatous organs, the heart muscle, and the endocrine glands. As a result, liver cirrhosis to hepatocellular carcinoma, diabetes mellitus (bronchial diabetes), dark skin pigmentation, secondary cardiomyopathy, and extensive endocrine disorders and arthropathy may develop. Clinical symptoms usually manifest after age 40, with male affected individuals showing symptoms earlier than females.

Haemochromatosis is one of the most common hereditary diseases with a frequency of 1:400 to 1:200. It is characterized by a pronounced iron overload of the organism, caused by increased iron absorption in the upper small intestine. The excess iron is deposited in various organs, especially in the liver, pancreas, heart muscle, joints and pituitary gland. This organ distribution determines the picture of haemochromatosis, which is characterized by hepatomegaly (enlarged liver), liver cirrhosis with increased risk of developing hepatocellular carcinoma, diabetes mellitus, cardiac muscle weakness, joint disease, impotence or amenorrhea and dark skin pigmentation ("bronze diabetes").

Iron is an essential micronutrient that is problematic for biological systems since it is toxic as it generates free radicals by interconverting between ferrous (Fe^{2 +}) and ferric (Fe³⁺) forms. Additionally, even though iron is abundant, it is largely insoluble so cells must treat biologically available iron as a valuable commodity. Thus, elaborate mechanisms have evolved to absorb, recycle and store iron while minimizing toxicity. Focusing on rarely encountered situations, most of the existing literature suggests that iron toxicity is common. A more nuanced examination clearly demonstrates that existing regulatory processes are more than adequate to limit the toxicity of iron even in response to iron overload. Only under pathological or artificially harsh situations of exposure to excess iron does it become problematic.

If iron overload or an imbalance of iron homeostasis occurs in the cytosol, reactive oxygen species (ROS) are formed due to the redox balance. The increase in Fe(II) ions in the cytosol simultaneously leads to an increase in oxygen species through the release of the electron:

Fe³⁺ + O₂ ----> e- -----> Fe²⁺ + ·O₂ -----> ROS ------- > Apoptosis of lipid layer

The complexation of iron-ions (Fe²⁺ or Fe³⁺) with a ligand with a high affinity for iron-ions can contribute significantly to preventing the formation of free reactive oxygen species.

Further, unphysiological iron overload (intra- and/or extracellular) will cause endothelial cell damage. There is an attachment and activation of inflammatory cells, especially granulocytes and in a later phase monocytic cells, which migrate into the tissue and release mediators, proteases and reactive oxygen species (ROS), which further increase the damage. Released mediators such as NO, endothelin, cyto- and chemokines, leukotrienes and thromboxane lead to prothrombotic changes in the endothelium as well as increased vasoconstriction, which together with microcirculatory disturbances triggered by endothelial cell damage can lead to renewed hypoxia in some areas. As this inflammatory component is a secondary process, it has to be significantly inhibited by reducing the damage induced by ROS generated though iron overload.

Various pathophysiological processes related to ROS development causing endothelial cell damage, which is interpreted partly as a cause and partly as a consequence of inflammatory processes and in the pathogenesis of which reactive oxygen species appear to play a significant role. New methodological approaches in physiology, cell biology and biochemistry have provided new insights into the mechanisms of ischemic damage and reperfusion damage, the targeted prevention of which is being expected to lead to a significant improvement in conservation. In recent years, the cold used to protect the organs has also turned out to be an independent damaging factor; none of the preservation solutions currently used is able to provide protection against this damage.

Hemochromatosis describes the form of iron storage disease, a quite frequently inherited disease. Due to a genetic defect, the body can no longer regulate the homeostasis of iron metabolism. In iron storage disease, the body absorbs more iron from food than it needs. One in 200 people in Germany is affected; an estimated 8 million German citizens carry iron storage disease, many of them without knowing it. Anyone affected can pass the disease on to their offspring. If the disease is not recognized, an overload of iron in the liver leads to cirrhosis. Likewise, the pancreas becomes inflamed, often leading to diabetes mellitus.

Diagnostics include the determination of the laboratory values ferritin and transferrin saturation, genetic testing and, in certain cases, liver biopsy. The treatment of choice is phlebotomy, only in the presence of contraindications treatment with the drug deferoxamine. The extent of iron overload and the timing of the start of phlebotomy therapy show a clear influence on the prognosis and occurrence of complications of haemochromatosis. These observations underline the importance of early diagnosis and consistent therapy in hemochromatosis. The initiation of a consistently performed phlebotomy treatment before the onset of diabetes mellitus and liver cirrhosis results in a normal life expectancy.

Haemochromatosis is caused by increased iron absorption in the upper small intestine. Since the body has no way of excreting excess iron, the excess iron is deposited in certain organs and leads to organ damage.

If left untreated, hemochromatosis leads to death. Treatment for hemochromatosis may include reducing iron levels by removing blood via bloodletting or iron chelating therapy, dietary changes and treatment to avoid complications of the disease. The goal of treatment is to reduce the amount of iron in the body to normal levels, prevent or delay organ damage from excess iron, and maintain normal amounts of iron throughout the lifetime.

Treatments that can be used to reduce iron levels in people with other types of hemochromatosis include removing blood (phlebotomy) or iron chelation. These treatments can prevent additional organ damage but typically do not reverse existing damage.

Parenteral administration is particularly indicated in the treatment of chronic iron overload, e.g. transfusion hemosiderosis (iron overload of the organism as a result of frequent blood transfusions), thalassemia major, sideroblastic anemia, autoimmune hemolytic anemia and other chronic anemias. This applies in particular to primary (idiopathic) haemochromatosis (abnormal, increased deposition of iron in the organism) in patients whose concomitant disease (e.g. severe anemia, heart disease, hypoproteinemia (reduced protein concentration in the blood)) should not be phlebotomized.

Further, excess iron may be removed by an iron chelation therapy. In some cases, for example patients who have anemia and iron at the same time and thus cannot donate phlebotomy, an iron chelation therapy may be the only way to treat the iron overload. Anemia with iron overload is prominent in several conditions such as sickle cell disease, thalassemia major, myelodysplasic syndromes (MDS), enzyme disorders, iron transport or storage disorders, and some forms of cancers. Patients with these conditions require repeated blood transfusions to survive. Each unit of blood used in transfusion contains about 250 milligrams of iron. The body cannot excrete iron, except in tiny amounts-about one milligram per day, which is sloughed off in skin or perspiration. Therefore, the excess iron is trapped in the tissues of vital organs, such as the anterior pituitary, heart, liver, pancreas and joints. When the iron reaches toxic levels, damage can result in diseases such as diabetes, cirrhosis, osteoarthritis, heart attack, and hormone imbalances. Hypothyroidism, hypogonadism, infertility, impotence and sterility can result from these hormone imbalances. The patient can experience symptoms of chronic fatigue, mood swings, loss of sex drive, confusion, and memory loss. If not addressed, excess iron can result in complete organ failure and death.

It is important to remove the unnecessary iron before damage can occur. Hence, there is a great demand for iron reduction being accomplished with chelation therapy. Chelation therapy is the removal of iron pharmacologically with an iron-chelating agent such as deferoxamine (*N*'-[5-(Acetyl-hydroxy-amino)pentyl]-*N*-{5-[3-(5-aminopentyl-hydroxy-carbamoyl)propanoylamino]pentyl}-*N*-hydroxy-butandiamid) or deferasirox (4-(3,5-bis(2-hydroxyphenyl)-1,2,4-triazol-1-yl)benzoic acid). Both of these products are manufactured by Norvartis Pharmaceuticals. Another chelating agent is deferiprone.

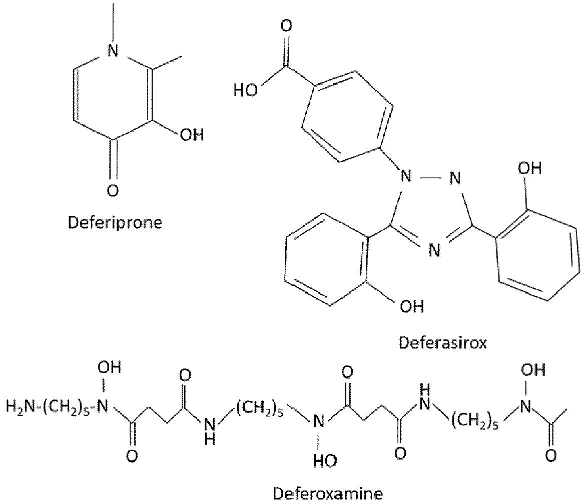

These drugs are specifically formulated to bind with iron so that the iron can be excreted in urine. Their clinical activity is influenced by the chemical and physiochemical properties. Deferiprone and deferoxamine are hydrophilic chelators that increase iron excretion and decrease iron absorption.

Deferoxamine, brand name Desferal, is not absorbed in the intestinal tract; therefore, this drug must be administered intravenously at an infusion center. Alternatively, the drug can be given subcutaneously, using a portable battery-operated infusion pump. Generally, the pump is worn at night, where slow infusion of the iron chelating agent is administered over a period of about eight hours, for the duration of four to six nightly infusions per week.

Deferoxamine works in two ways: firstly, it binds tightly to iron, and as a result the corresponding iron complex, known as ferrioxamine, is formed. This can be excreted by the body (unlike free iron). Secondly, it very quickly reduces the tissue-damaging effect of iron by preventing the formation of free radicals.

Deferoxamine mainly binds the free iron that is released when the red blood cells are broken down. The iron is then excreted in the urine. To a lesser extent, deferoxamine binds to the iron that is released when storage iron is broken down in the liver cells. This compound is then also removed from the body in the stool. Due to its molecular size and its hydrophilic character, however, deferoxamine is not able to penetrate into other body cells such as heart muscle cells and liver cells and bind iron there. Thus, treatment with deferoxamine alone is not sufficient in patients with heart problems. In addition to reliability in the recommended use by the patient, the success of treatment with deferoxamine also depends on the extent of iron overload as well as sufficient vitamin C levels in the body.

Further, due to toxicity implications, the treatment with deferoxamine is restricted to cases with a heavily iron-load, to iron-loaded patients with a serum ferritin higher than 500 pg/L. Toxic side effects such as fatal renal, liver, and bone marrow failure as well as renal toxicity, skin rashes and gastric intolerance have been reported in iron-loaded patients treated with deferoxamine. Thus, patients being treated with deferoxamine have to be monitored frequently.

Deferiprone, brand name Ferriprox, is different from deferoxamine in that deferiprone forms lipophilic iron complexes and can be taken orally.

The same holds for deferasirox. Both chelating agent can increase the iron absorption.

The pharmacological, toxicological, biochemical and iron-binding properties of the chelators discussed above are described in literature, e.g. in "Efficacy and safety of iron-chelation therapy with deferoxamine, deferiprone, and deferasirox for the treatment of iron-loaded patients with non-transfusion-dependent thalassemia syndromes" in Drug Design, Development and Therapy, January 29, 2016; 2016:10 p. 465-481. However, drug therapies including the known chelators are not efficient enough for monotherapy and/or are associated with significant side effects when used for prolonged periods.

One main drawback of the chelators with a high molecular weight like deferoxamine is, that these chelators show a very low intracellular bioavailability. Hence, the chelators may bind iron only in the extracellular regions while they can't pass the lipophilic cell membrane. This is disadvantageous, since it is not possible to remove intracellular iron. Thus, excess iron in heart and liver, which are the organs suffering the most from an iron excess, aren't removed by iron chelating agents like deferoxamine.

### Object of the invention

It is thus subject of the invention to provide a preparation for an efficient intracellular complexation of iron, especially for treatment of pathological iron overload, without showing the drawbacks known from the state of the art.

This object is solved by the subject matter of the independent claims. Preferred embodiments and variants are subject of the depending claims.

Preparation for reduction of pathological iron overload in human body, especially for treatment of hemochromatosis, is disclosed, wherein the preparation comprises a hydroxamic acid derivate.

The hydroxamic acid derivate builds a stable complex with iron ions. Here, the structural element of hydroxamic acid is of particular importance as a suitable ligand for iron.

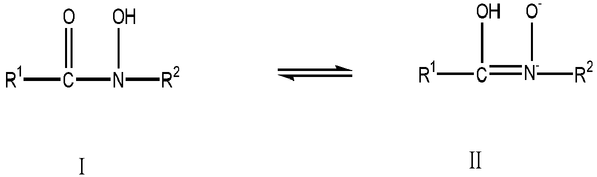

The hydroxamic acid derivate comprises one hydroxamic acid group. The hydrogen atom on the hydroxamic acid carbon is aryl- or alkylaryl substituted, wherein said substituent may comprise hydroxyl, alkoxy or amino groups. Preferably, the hydrogen atom on the hydroxamic acid carbon is substituted by phenyl derivates or benzyl derivates.

The structural design of the hydroxamic acid derivates according to the invention has been optimized in view of the metabolic pathways and thus shows tailored properties.

Preferably, the nitrogen atom is alkyl substituted. Compared to an unsubstituted derivate, said embodiments are generally more effective in view of their iron chelating effect. This effect may be caused by the positive inductive effect of alkyl-substituents. However, introducing an electron-withdrawing group as a substituent, like e.g. a carbonyl group, on the nitrogen atom leads to a decrease of effectiveness, although these compounds may still form iron complexes.

Preferably, the hydroxamic acid derivate has the following structure 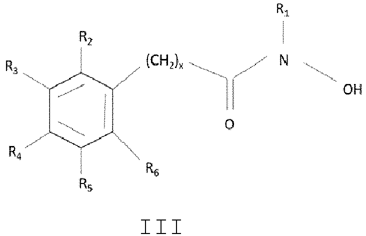 with x = 0 or 1, R₁= H, alkyl, preferably alkyl with at most four Carbon atoms, more preferably methyl,
R₂, R₃, R₄, R₄, R₅, R₆ = H, OH, alkoxy, preferably alkoxy with at most four carbon atoms, more preferably methoxy.

The hydroxamic acid derivates according to the invention are relatively small and lipophilic compared with iron chelating compounds known from the state of the art like e.g. deferoxamine. According to one embodiment, the hydroxamic acid derivate comprises alkyl and/or alkoxy substituents with not more than four carbon atoms. Preferably, the alkoxy and/or alkyl substituents comprise one carbon atom. This reduces the size and/or molecular mass of the hydroxamic acid derivate.

Surprisingly, although longer alkyl or alkoxy substituents enhance the lipophilia of the hydroxamic acid and show a stronger inductive effect, thereby enhancing the activity of the hydroxamic acid, a reduced size and/or molecular weight is advantageous in view of the effectivity of the iron reduction.

According to one embodiment, the molecular mass of the hydroxamic acid derivate is less than 560 g/mol, preferred less than 370 g/mol and more preferred less than 270 g/mol. Preferably, the molecular mass of the hydroxamic acid derivate is at least 130 g/mol and/or less than 320 g/mol.

According to one embodiment, the preparation comprises at least one hydroxamic acid derivate according to formula (III), wherein at least two of the substituents R2, R3, R4, R5, and R6 comprise alkoxygroups. Since alkoxygroups enhance the electron density, the effectiveness in view of iron complexation and thus in view of reducing the intracellular iron content may be enhanced.

Preferably, the n-octanol-water partition coefficient K_{OW} is at least 0, more preferred at least 1, most preferred at least 2. The n-octanol-water partition coefficient K_{OW} is a partition coefficient for the two-phase system consisting of n-octanol and water. K_{OW} serves as a measure of the relationship between lipophilicity and hydrophilicity of a substance. Substances which are more soluble in water than in fat-like solvents show a small value. The value decreases with the lipophilicity of a substance. K_{OW} values provide a good estimate how a substance is distributed within a cell between the lipophilic biomembranes and the aqueous cytosol. Since the disclosed hydroamine acid derivates show a relatively high K_{OW}, they many pass the cell membranes and thus may enter the intracellular space.

Due their small molecule size and their lipophilic character, the hydroxamic derivates according to the invention may pass the cell membranes and thus possess optimal intercellular bioavailability. Hence, a rapid intracellular availability can be achieved.

Hence, the hydroxamic acid derivates are in particular suitable to reduce the free iron in general but even more to reduce the free iron in the cytosolic compartments, thereby preventing destruction of cell-membranes due to ROS development.

In iron complexes of hydroxamic acid derivates according to the invention, the hydroxamic acid functionality provides the electrons for building the complex. Moreover, due to the chelating effect, complex-forming tendency increases with number of hydroxamic acid functionalities in the ligand. This enables the formation of strong iron complexes inside the cells.

Surprisingly, especially hydroxamic acid derivates with only one hydroxamic acid functionality per molecule, in particular hydroxamic acid derivates according to formula III, show a high effectiveness in view of reducing intracellular iron. This may be explained with the inductive effect of the aryl substituent on the carbon atom of the hydroxamic acid group, which may activate the remaining hydroxamic acid group. Hence, hydroxamic acid derivates according to formula III may build iron complexes in a sufficient manner. According to one embodiment, the hydroxamic acid derivate - iron complex has a constant log β in the range of 15 to 30. In a preferred embodiment, the constant log β is smaller than 25. Preferably, the constant log β is in the range of 18 to 24. This is advantageous, since it allows the release of the iron and building of new iron complexes in the presence of other ligands in the extracellular space like e.g. iron binding proteins. This rearrangement of iron complexation releases the hydroxamic acid derivate, which can again enter the intracellular space to bind again to iron.

Further, hydroxamic acid groups are relatively polar groups. Hence, increasing the number of hydroxamic acid groups in the molecule leads to a decrease of the K_{OW}-value.

Preferably, the preparation comprises at least one of the following hydroxamic acid derivates and/or salts thereof:

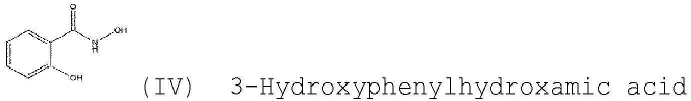

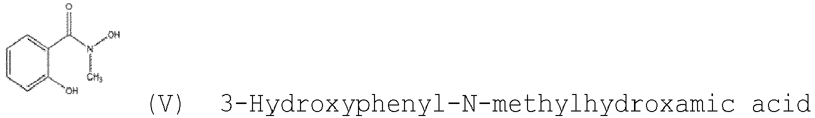

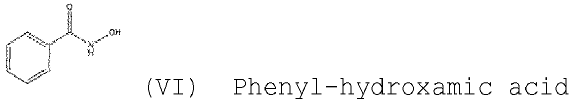

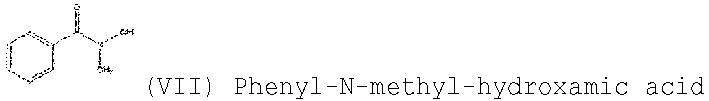

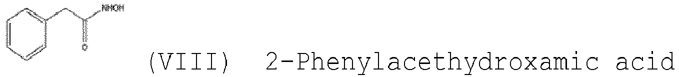

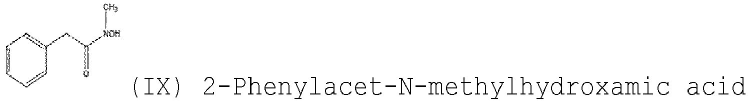

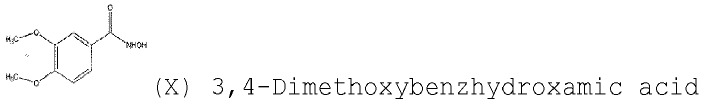

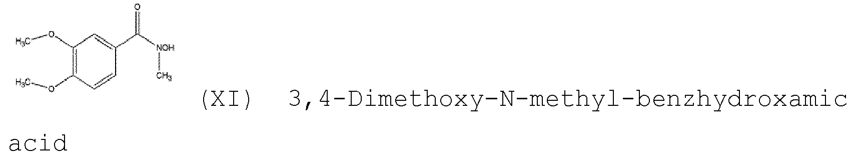

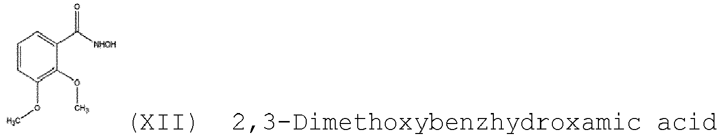

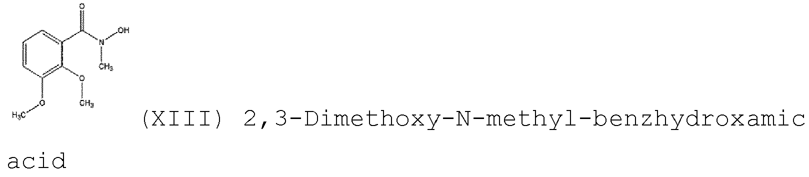

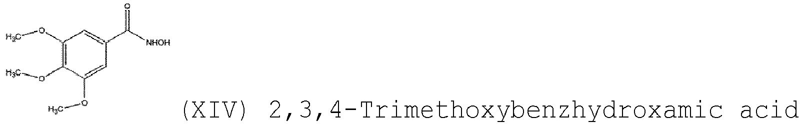

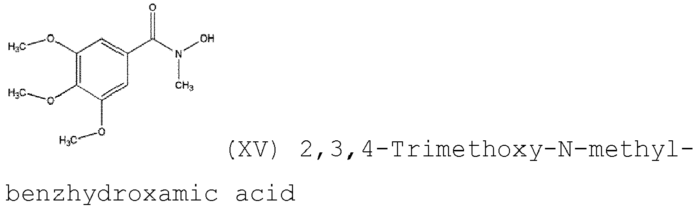

The principal mode of iron reduction can be described as such: The hydroxamic derivates according to the invention pass through the cell membrane and enter the intracellular space. Here, the hydroxamic derivates form an iron complex. The iron complex reaches the extracellular space. The extracellular space may comprise further compounds for building iron complexes, which may not enter the intercellular space, but compete with the hydroxamic acid derivates. In case said iron complexes show a higher equilibrium constant, i.e. the iron complex forms with this ligands show a higher stability, than the hydroxamic acid derivate according to the invention, the hydroxamic acid derivate is released from the iron-complex and is again available for complex building. According to equilibrium constant, the ligand will be released from the extracellular space into the intracellular space to act again intracellular. Due to this circuit, one can reduce the applied doses of the hydroxamic acid derivate, thereby avoiding severe side effects and achieving a high tolerability of the preparation.

The further iron ligands in the extracellular space may be body's own substances such as proteins. According to one embodiment, the further iron ligand may be part of the preparation. Preferably, the preparation comprises deferoxamine in addition to the hydroxamic acid derivate.

The hydroxamic acid derivates according to the invention have low toxicity and possess optimal intracellular bioavailability. After transfer into liver and/or gut, the elimination of iron follows the intestine rout. Preferably, the hydroxamic acid derivate has a LD₅₀-dose of at least 100 mg/kg, preferably of at least 200 mg/kg.

The preparation can be administered oral or parenteral. Preferably, the preparation is administered oral. This application method is much more comfortable for the patient and thus enhances live quality as well as compliance. The single dose of the preparation comprises preferably 5 to 100 mmol hydroxamic acid derivate.

In an alternative embodiment, the preparation is administered intravenously. According to one embodiment, the preparation is administered with a unit dose of 5 to 50 mmol hydroxamic acid derivate.

The hydroxamic acid derivate can be synthesized from the corresponding carboxylic acid esters or corresponding carboxylic acid chlorides. In a suitable solvent, the corresponding carboxylic acid esters are reacted with the corresponding N-alkyl-substituted hydroxylamine, the reaction being base-catalyzed by bases like e.g. NaOMe, K₂CO₃ or CaO. Suitable solvents are e.g. lower alcohols, dimethyl formamide (DMF) or tetrahydrofurane (THF).

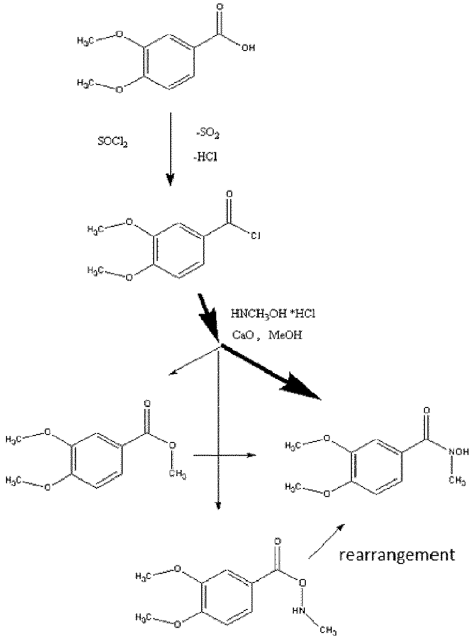

Following, the invention is described by exemplary embodiments.

### Toxicology

### 1. Acute toxicology of 2,3-dimethoxy-N-methylbenzhydroxamic acid (XIII) following intra venous administration of a single dose.

In the preliminary phase, 1 male and 1 female rat were dosed at 200 mg/kg and observed for a period of 7 days. No mortality and no clinical signs were observed. A further group of 1 male and 1 female animals was dosed at a dose level of 300 mg/kg. Clinical signs observed following dosing were pronation, reduced activity and ataxia. Recovery occurred approximately 4 hours after dosing.

In the main phase of the study, 1 group of 5 male and 5 female animals was dosed at 300 mg/kg and observed for a period of 14 days. No mortality occurred. Clinical signs observed following dosing were piloerection and reduced activity. Recovery occurred by Day 3.

Body weight losses were observed in the majority of the animals on Day 2. Body weight changes at the end of the study (Day 15) were in the normal range for this strain and age of animals.

No abnormalities were observed at necropsy examination performed in treated animals at the end of the observation period.

These results indicate that 2,3-dimethoxy-N-methylbenzhydroxamic acid (XIII) has a slight toxic effect in the rat following intravenous administration of a single dose at a level of 300 mg/kg body weight Therefore, this dose level is considered reasonably tolerated.

### 2. Gene toxicity of 3,4-dimethoxy-N-methylbenzhydroxamic acid (XI)

The in vitro Micronucleus Assay (MNA) is a mutagenic test system for the detection of chemicals that induce the formation of small membrane-bound DNA fragments (micronuclei (MN)) in the cytoplasm of interphase cells. The MNA, used for regulatory purposes measures formation of chromosomal changes following DNA damage induced by the compounds under test, and is used to predict the genotoxic potential of pharmaceuticals, industrial chemicals, food additives and cosmetic ingredients.

MN originate from chromosome fragments or whole chromosomes that are not included in the main daughter nuclei during nuclear division. They reflect chromosome damage and may thus provide a marker of genotoxicity and even early-stage carcinogenesis. The most commonly used method in mammalian cells is the cytokinesis-block micronucleus (CBMN) assay. In the CBMN assay, MN are scored after a single cell division using binucleated cultured cells (accumulated using cytochalasin B) to eliminate the confounding effect of altered cell division kinetics on the MN index.

The formation of MN is a consequence of chromosomal breakage and/or spindle-fiber dysfunction induced by clastogens and/or aneuploidogens. The present study was performed in accordance with the OECD 487 and under GLP requirements.

Under the OECD 487 recommendations, LK 614 was analyzed in micronucleus test at least 3 selected concentrations (Paragraph 28 and 29). DMSO controls were run concurrently for each experiment. Test item together with appropriate reference items (MMC and CP) were tested both with and without metabolic activation:
- +S9 short treatment (the final concentration of 1% v/v S9)
- -S9 short treatment
- -S9 extended exposure

The Giemsa-stained slides were analyzed on light microscope using criteria defined by Fenech *et al.* (2003) for scoring micronuclei. Micronuclei were scored in binucleated cells, in which both nuclei were of similar size and intensity and the micronuclei size was ≤0.33 the size of the main nuclei, and of similar intensity as the main nuclei (Fenech et al. Mutation Research, Vol. 534, 2003). Micronucleus frequency was analyzed in at least 2000 binucleate cells per concentration and control.

MNA test result revealed no genotoxicity of test item, LK 614, in any of tested experimental conditions. LK 614 tested in system with and without metabolic activation in tested concentrations did not exhibit statistically significant increase in micronucleus frequency per culture compared with the concurrent negative control (Chi-square test with Yates' correction, P>0.05).

A significant concentration-related increase in frequency of MN was not observed in cultures treated with compound named LK 614 (Chi-square test for trend, P>0.05).

MN formation was significantly induced in CHO-K1 cells compared to the control following exposure to reference items (MMC and CP) at indicated concentrations (P<0.05, Table IV). The number of CHO-K1 cells with MN increased in an MMC/CP exposure concentration-dependent manner (P<0.05). Results obtained for reference items (MMC and CP) demonstrated reproducibility and sensitivity of system used to analyze genotoxic potential of items.

In order to assess genotoxic potential. CHO-K1 cells were exposed to tested item, LK 614, and appropriate reference items in system with (+S9) and without (-S9 short and extended treatment) an exogenous metabolic activation. Statistical analysis of the MN frequency and binucleate cells with MN was performed using the Chi-square test with Yates' correction. To examine the dose-response relationship in frequencies of the micronuclei Chi-square test for trend was performed.

None of tested concentrations of test item, LK 614, exhibits a statistically significant increase in MN frequency compared with the concurrent negative control (P>0.05). Chi-square test for trend revealed no dose-related increase in MN frequency (P>0.05).

Results for positive controls, mitomycin C and cyclophosphamide, demonstrated reproducibility and sensitivity of test system.

In summary, the present research has demonstrated that test item, compound named LK 614, did not produce dose-dependent genetic toxicity in the CHO-K1 cells.

## Claims

1. Preparation for reduction of pathological iron overload in human body, especially for treatment of hemochromatosis, the preparation comprising a hydroxamic acid derivate, wherein the hydroxamic acid derivate builds a complex with iron ions, wherein the hydroxamic derivate comprises a hydroxamic acid group and wherein the hydrogen atom on the hydroxamic acid carbon is aryl- or alkylaryl substituted, wherein said substituent may comprise hydroxy, alkoxy or amino groups.

2. Preparation according to claim 1, wherein the hydroxamic acid derivate (III) has following structure 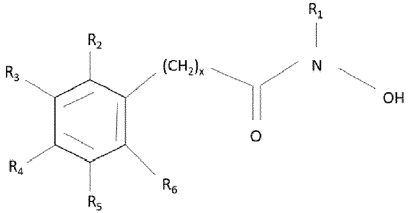 with x = 0 or 1, R₁ = H, alkyl, preferably alkyl with at most four carbon atoms, more preferably methyl, R₂, R₃, R₄, R₅, R₆ = H, OH, alkoxy, preferably alkoxy with at most four carbon atoms, more preferably methoxy.

3. Preparation as claimed in any one of claims 1 or 2, wherein the preparation comprises at least one of the following hydroxamic acid derivates and/or the salts thereof:
phenylacetydroxamic acid, phenylacet-N-methylhydroxamic acid, dimethoxybenzohydroxamic acid, 2,4-dihydroxybenzohydroxamic acid, 2,3-dihydroxybenzohydroxamic acid, 3,4-dimethoxy-M-methylbenzohydroxamic acid, 4-hydroxy-3-methoxybenzohydroxamic acid, 2-hydroxy-3-methoxybenzohydroxamic acid.

4. Preparation as claimed in claim 2, wherein at least two of the substituents R₂, R₃, R₄, R₅ and R₆ comprise alkoxygroups, preferably methoxygroups.

5. Preparation as claimed in any of the preceding claims, wherein the hydroxamic acid derivate has a n-octanol-water partition coefficient, *K*_{ow} of at least 0, preferably at least 1, more preferable at least 2.

6. Preparation as claimed in any of the preceding claims, wherein the hydroxamic acid derivate comprises only one hydroxamic acid group and/or is a bidentate ligand.

7. Preparation as claimed in any of the preceding claims, wherein the hydroxamic acid derivate has a molecular weight less than 560 g/mol, preferably less than 370 g/mol, more preferably less than 270 g/mol and/or the molecular weight is in the range between 130 and 320 g/mol.

8. Preparation as claimed in any of the preceding claims, wherein the complex formed between the hydroxamic acid derivate and iron build complex formed has a complex formation constant log β which is smaller than 30, preferably smaller than 25 and/or lies in the range from 15 to 30, preferably in the range from 18 to 24.

9. Preparation as claimed in any of the preceding claims, wherein the complexes formed between the hydroxamic acid derivate and iron are build intercellular.

10. Preparation as claimed in any of the preceding claims, wherein the preparation is administered orally or parenteral, preferably orally.

11. Preparation as claimed in any of the preceding claims, wherein the preparation is administered orally with a unit dose of 5 to 100 mmol hydroxamic acid derivate.

12. Preparation as claimed in any of the preceding claims 1 to 10, wherein the preparation is administered intravenously with a unit dose of 5 to 50 mmol hydroxamic acid derivate.

13. Preparation as claimed in any of the preceding claims, wherein the hydroxamic acid derivate has a LD₅₀-dose of at least 100 mg/kg, preferably of at least 200 mg/kg.

14. Preparation as claimed in any of the preceding claims, wherein the preparation comprises in addition to the hydroxamic acid derivate a further complexing agent for building of iron complexes, preferably deferoxamine.
